# EUROPEAN PATENT APPLICATION

(11) **EP 3 037 546 A1**
(43) Date of publication of application: **29.06.2016**
(21) Application number: 14786549.7
(22) Date of filing: 22.08.2014
(51) Int. Cl.: C12Q 1/68

(54) **MOLECULAR TAG CONTAINING DNA MOLECULES AND PROCESS FOR MARKING AND IDENTIFYING THE TAG**

(30) Priority: 23.08.2013 PT 10712413
(71) Applicant: Universidade de Aveiro, Aveiro 3810-193 (PT)
(72) Inventor: MARCIAL GOMES, Newton Carlos, P-3810-193 Aveiro (PT)
(74) Representative: Bairrão, Isabel
(86) International application number: PCT/PT2014/000056
(87) International publication number: WO 2015/026254

(57) **Abstract**

The present invention relates to a molecular label, and to a process for marking different materials and liquid substances by using chimeric deoxyribonucleic acid molecules (DNA) for label formulation, and to the use of polymerase chain reaction techniques (PCR) and denaturing gradient gel electrophoresis (DGGE) for the identification of the label.

The molecular label for marking a substrate herein described comprises:
a mixture of a plurality of DNA molecules, each comprising a GC-clamp DNA fragment as an identifier segment and a microbial DNA fragment as a encoder segment,
wherein at least two of said microbial DNA fragments derive from different microorganisms;
and a carrier for supporting said mixture.

Thus, the present invention falls within the field of traceability and authentication of different materials with application in any kind of activity wherein the use of tracking or certification of origin is required.

SEQUENCE LISTING

<110>  UNIVERSIDADE DE AVEIRO

<120>  ETIQUETA MOLECULAR CONTENDO MOLÉCULAS DE ADN E PROCESSO DE
       MARCAÇÃO E IDENTIFICAÇÃO DA ETIQUETA

<130>  10615IB/mcd

<140>  PCT/PT2014/000056
<141>  2014-08-22

<150>  107124
<151>  2013-08-23

<160>  7

<170>  PatentIn version 3.5

<210>  1
<211>  57
<212>  DNA
<213>  Escherichia coli

<400>  1
cgcccggggc gcgccccggg cggggcgggg gcacgggggg aacgcgaaga accttac          57

<210>  2
<211>  24
<212>  DNA
<213>  Escherichia coli

<400>  2
cggtgtgtac aaggcccggg aacg                                              24

<210>  3
<211>  20
<212>  DNA
<213>  Escherichia coli

<400>  3
ggccagtgaa ttgtaatacg                                                   20

<210>  4
<211>  20
<212>  DNA
<213>  Escherichia coli

<400>  4
cacaggaaac agctatgacc                                                   20

<210>  5
<211>  20
<212>  DNA
<213>  Escherichia coli

<400>  5
taatacgact cactataggg                                                   20

<210>  6
<211>  18
<212>  DNA
<213>  Escherichia coli

<400>  6
atttaggtga cactatag                                                    18

<210>  7
<211>  40
<212>  DNA
<213>  Escherichia coli

<400>  7
cgcccggggc gcgccccggg cggggcgggg gcacgggggg                            40

## Description

### Technical field of the invention

The present invention relates to a molecular label and to a process for marking different materials (solid or liquid) from the use of molecular labels containing chimeric deoxyribonucleic acid molecules (DNA).

Thus, the present invention falls within the field of traceability and authentication of materials with application in any type of activity wherein the use of traceability or certification of origin is required (ex. food, pharmaceutical, veterinary and agricultural industries) or in any type of activity wherein the use of traceability or certification of origin is required. One of the aims of the present invention is to identify and trace different (biological and non-biological) materials and liquid substances.

### Background of the Invention

With the increasing market globalization the registration of information concerning the origin of the goods and the circumstances in which they are produced has become ever more important. Therefore, the use of a traceability and identification system for the goods is of fundamental importance to ensure the origin, quality and safety of a particular good. Furthermore, the implementation of a traceability system may add value to the product. Primarily, it is essential for the traceability methods to ensure at least two basic pieces of information about the goods: identification and certification of origin.

The systems currently used are mostly based on the use of adhesive labels with or without barcodes and electronic labels with radiofrequency identification (RFID). The first patent based on barcodes was published in 1952 (US 2612994). The electronic labels are mostly used in supply chains, at an industrial level or for high-value differentiated products. The first patent for an RFID label was granted in 1973 (US 3713148).

In 2004, for the first time, Beda M. Stadler has published a patent document claiming the use of DNA sequences to store information, which may be used to mark objects or non-human organisms (US 20040043390 A1).

Such information is in turn obtained from DNA molecules (previously synthesized containing the desired information in code form) through the use of sophisticated technologies.

### General Description

The present invention relates to a process for marking different materials and liquid substances by using molecular labels containing chimeric deoxyribonucleic acid molecules (DNA).

A plurality of chimeric DNA molecules may be used in the preparation of the label. The label may be placed onto the surface of different materials or liquid substances. The reading of the label can be made through polymerase chain reaction (PCR) and denaturing gradient gel electrophoresis (DGGE) techniques. The set of molecules detected in a label provides relevant information to control the product as a barcode form. The labelled material can be associated with primary and industrial products.

Therefore, the present invention falls within the field of traceability and authentication of different materials with application in any type of activity wherein the use of traceability or certification of origin is required.

However, currently existing labels can be forged, falsified, replaced by other labels or be simply removed. The present invention aims at overcoming such limitations by marking the products with molecular labels synthetized from microbial DNA wherein the mixture does not exist directly in nature. Molecular labels may thus be used as an alternative to or together with the technologies previously mentioned in the marking of products in general.

The present invention enables that fragments from any source of microbial DNA (for example biological and environmental samples) may be used in the preparation of the model chimeric DNA molecule (DNA used as a model for the formulation of molecular label), without the need of any type sequencing technology. Therefore the DNA molecules used in this invention do not store information as previously described (US 20040043390 A1).

The present invention relates to a molecular label and a process for marking different materials (either biological, non-biological or liquid substances) by using molecular labels containing chimeric DNA molecules. Thus, the presented solution allows for a scalable code based on natural sources, but which does not exist in nature as such, and yet is easy and quick to identify (for example to determine only a random sample in a given batch of products).

In the present invention the code is rated as easily scalable insofar as the combination possibilities make it possible to apply a molecular label containing a specific code to each product. In a preferred embodiment, in a hypothetical case wherein the molecular label to be applied consists of 10 bands (10 types of model chimeric DNA molecules), and for which purpose there is a set of 20 types of model chimeric DNA, it is possible to obtain 184756 different codes.

This without taking the possibility of adding variations on the intensity of each band used in the molecular label into consideration. If we add to this fact, the possibility of using DNA from different microorganisms, the molecular label described in the present invention is hardly forged.

Via DGGE a band profile is obtained, whose presence/absence and intensity are capable of acting as codifications basis (analogously to the traditional barcode form) of the molecular label. This codification form of the present invention is designated as a barcode.

In addition to increasing the combination probabilities, the present invention also makes the process of creating and formulating molecular labels much easier, while reducing costs inherent to DNA label reading and synthesis. The simplicity of the reading methodology of the present invention further allows the possibility of identifying and authenticating the product *in* situ through the use of portable molecular biology equipment. A further advantage is the higher reliability. Given that DNA molecules are chimeric, it is hardly possible for these to occur naturally.

One aspect of the present invention describes a molecular label to mark a substrate comprising:
a mixture of a plurality of DNA molecules (or chimeric DNA molecules), each comprising a GC-clamp DNA fragment (GC-clamp SEQ. ID. No. 7) as an identifier segment and a microbial DNA fragment as a encoder segment,
wherein at least two of said microbial DNA fragments derive from different microorganisms;
and a carrier for supporting said mixture.

Model chimeric DNA molecule means each DNA molecule comprising a GC-clamp DNA fragment as an identifier segment and a microbial DNA fragment as a encoder segment.

In another embodiment said DNA molecules were obtainable separately.

In another embodiment the microbial DNA fragments of the molecular label of the present invention can have a size that varies from 200-1800 pb (pair of basis).

In another embodiment the molecular label fragments of the present invention may have the same number of pair basis.

In another embodiment the molecular label of the present invention may further comprise primers selected from the following list: SEQ. ID NO.1; SEQ ID NO.2; SEQ.ID NO.3; SEQ. ID NO.4; SEQ.ID NO.5; SEQ. ID NO.6.

In another embodiment the concentration of each of said model chimeric DNA molecules is variable, wherein a certain density conferred by the respective DNA concentration.

In another embodiment the molecular label of the present invention may be in dehydrated, lyophilized or liquid form.

In another embodiment of the molecular label the substrates to mark can be solid or liquid.

In another embodiment the carriers for solid substrates of the molecular label of the present invention may be selected from the following list: epoxy resins, dyes, carrier particles, nanoparticles, phospholipid membranes, among others.

In another embodiment the carriers for liquid substrates of the molecular label of the present invention may be selected from the following list: aqueous solutions, solutions comprising ethanol, solutions comprising acetone, among others.

Another aspect of the present invention, are labelled objects with the labels described in the present invention. The labelled objects may be food, medicines, paper, documents, leather, clothing, pens, watches, jewellery, an electronic article, plastic container and labels.

Another aspect of the present invention relates to a process
- for applying the molecular label described in the preceding claims onto the substrate to mark comprising the following steps:
- synthesis of each chimeric DNA molecule - model - from a plurality of primers and gene fragments selected from one or more microbial DNA molecules, wherein each DNA molecule contains GC-clamp DNA fragment (GC clamp - SEQ ID No. 7) as an identifier segment and a microbial DNA fragment as encoder segment;
- mixture of each DNA molecule obtained;
- production of at least one molecular label comprising a plurality of DNA molecules, with a certain density conferred by the respective DNA concentration;
- marking of the substrate with at least a molecular label.

In another embodiment of the marking process of the present invention said DNA molecules may be obtained separately, in particular, by the cloning step in *Escherichia Coli* and preparation of genomic libraries containing different types of inserts.

In another embodiment of the marking process of the present invention the synthesis of at least one of said DNA fragments comprises the insertion of specific primers to the sites near the addition of specific primers on sites adjacent to the region of plasmid insertion. Preferably said primers may be selected from the following list: SEQ ID NO.1; SEQ ID NO.2; SEQ. ID NO.3; SEQ. ID NO.4; SEQ. ID NO.5; SEQ. ID NO.6.

In another embodiment of the marking process of the present invention the molecular label obtained may be dehydrated, lyophilized or remain in solution or in nanoparticle form.

In another embodiment of the marking process of the present invention the marking of the substrate/material may be carried out by sprinkling, droplet or micro droplet deposition, impregnation with adhesive and/or resiny substances.

In another embodiment of the marking process of the present invention the label described may further comprise a phospholipid membrane.

In another preferred embodiment, the plurality of DNA molecules may be used in the preparation of the label - i.e. in the synthesis of a model chimeric DNA molecule, it is necessary to initially amplify through polymerase chain reaction (PCR) target DNA fragments (example: the gene 16S rRNA) from the DNA extracted from an environmental sample, from a microbial species or a plurality of microbial species naturally present in the sample. For such purpose, specific primers are required for the target gene (example: primers F968-GC/ SEQ ID NO.1 and L1401 / SEQ ID NO.2 for the 16S rRNA gene). Whereas one of the primers must contain the GC-clamp at the 5' terminal (SEQ. ID NO.7). The PCR-amplified fragments obtained are then connected to a vector (plasmid) and cloned. A PCR is then performed to the insert (DNA fragment inserted into the plasmid) of each clone previously isolated with specific primers for the sites adjacent to the insertion region of the plasmid (primers D1 - SEQ. ID NO.3 and R1 - SEQ. ID NO.4). A second PCR is then carried out to the PCR product obtained this time using primers D2 - SEQ. ID NO.5 and R2 - SEQ. ID NO. 6. The PCR product of each insert is then analysed in a denaturing gradient gel electrophoresis (DGGE) for determining the mobility thereof. This step allows identifying clones carrying inserts with correct sizes and with different mobility in DGGE. One or more clones carrying different inserts are then selected for subsequent use in the formulation and production of the molecular label. For the selected clones, PCR is again performed to the insert using the specific primers D1 (SEQ. ID NO.3) and R1 (SEQ. ID NO.4). In this step the production of different chimeric DNA molecules is begun at the desired concentrations for the formulation of the molecular label. At this stage the PCR product of the insertion is then herein referred to as model chimeric DNA molecule. The PCR product of at least one insert is used for the formulation of the molecular label, the more inserts are added to the label composition more bands of DGGE will be present in the label. The DNA amount or concentration (PCR product) of each insert will also influence in the intensity of each band.

Another aspect of the present invention describes a process of identification and/or traceability of different materials comprising the detection of at least one molecular label described herein.

Another embodiment of the process of identification and/or tracking described in the present invention further comprises the step of comparing at least one molecular label with the profile of a reference sample (or DNA molecular label) of the marked material.

In another embodiment the added amount of each type of the chimeric DNA molecule / fraction in the molecular label is also used as information - for example the intensity of the bars (bands), during the reading process thereof. Therefore, the detection and reading of the label of the present invention is highly specific thereby making false copies or forging difficult, allowing that each label generated to serve as a guarantee seal for numerous substrates/materials/products.

Carrier - In the present invention carrier is used as a generic term for substances that could be used in the application and formulation of the molecular label with the purpose of increasing its volume or mass. In the present invention these substances may act on the application process, on the stability and protection of the label (example: adhesive substances, resins, dyes, aqueous solutions and carrier substances or particles).

Substrate/Material - in the present invention substrate is assumed to refer to any object or material in which the molecular label may be added (example: food, medicines, objects, pens, watches, paper; jewellery, electrical or electronic objects).

The label may be placed in the surface of various materials (either biological or non-biological), added in several food products or liquid substances. The detection of DNA molecules (identification of the label) is made through the techniques of polymerase chain reaction (PCR) and in denaturing gradient gel electrophoresis (DGGE). These techniques allow detecting each chimeric DNA molecule used in the preparation of the label in the form of band profiles (or barcode). The product (or material) marked with the label may be related with primary and industrial products (or different materials), whose origin and identification is unknown or intended to be confirmed. Thus, the present invention falls within the field of traceability and identification of various materials and liquid means with application in several industries (ex. food, pharmaceutical, veterinary and agricultural industries) or any type of activity in which the use of traceability or certification of origin is required.

Through the use of the molecular label, the present invention aims at providing safer and more reliable information about a particular product, thus avoiding that the information regarding the identification and origin of the goods is removed or forged during the different phases of production, processing, distribution and/or commercialization. Therefore, one of the objects of present invention is to provide a label and a marking process of various materials (either biological or non-biological) through the use of molecular labels containing chimeric - DNA molecules, of microbial origin.

### Technical advantages of the present invention

Initially the synthesis of DNA barcodes involves the creation of the model chimeric DNA molecule. This molecule is comprised by DNA copies of natural origin and DNA of synthetic origin that will be subsequently produced in large quantity through PCR technic. Most of the chimeric DNA molecule is synthesized from another DNA molecule easily obtained from the environment, from pure cultures or mixtures of environmental microorganisms. A DNA fragment of any type or microbial origin can be used for preparation of the model chimeric DNA molecule provided that said microbial DNA fragments have different mobility in DGGE. Furthermore, the use of natural DNA fragments in the synthesis of the chimeric molecule reduces costs associated with the synthesis of DNA molecules by specialized companies.

The technology of the present invention produces non-codified chimeric DNA molecules non-existent in nature and without addition of DNA atypical chemicals (example. fluorochromes). Therefore, they do not provide encoded information that might endanger the environment or substances of unknown effect on human health. Thus, this type of labels may be used in food, pharmaceutical and/or cosmetic products.

After the creation of model chimeric molecules, the large-scale label synthesis, is of low cost and does not involve cloning procedures. The molecular label can be produced in large quantities without the need for sophisticated and expensive apparatus. The use of a thermocycler and respective reagents is sufficient.

The addition of a GC clamp and/or primers allows the reading of the molecular label to be very easy and does not require sophisticated and expensive equipment and does not imply the sequencing of the molecule. The label reading can be done directly from the sample collected by PCR-DGGE, using for this purposes: a thermocycler, a denaturing gradient gel electrophoresis (DGGE) and respective reagents. Hence the reading of the labels is fast and cost-effective.

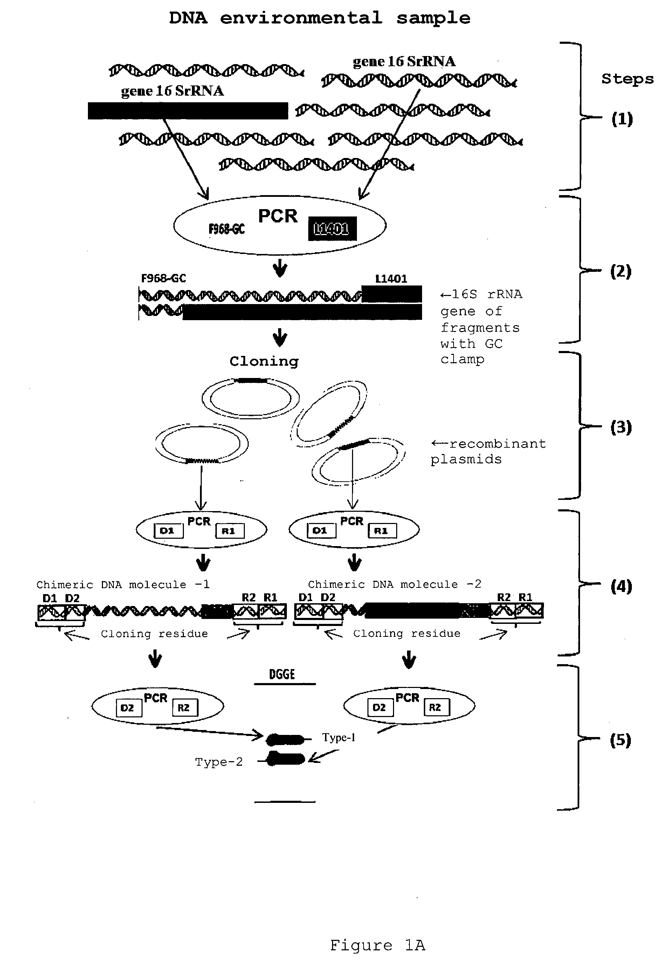

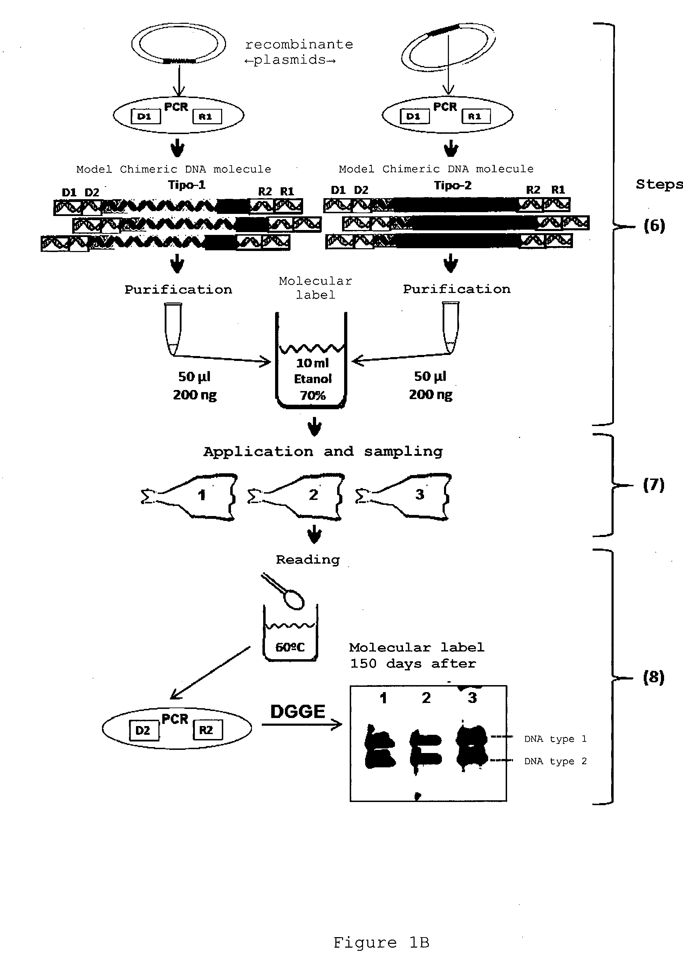

In one embodiment of the present invention the label reading is carried out from the identification of the number and intensity of the bars (DGGE bands) (Figure 1) rather than from the nucleotides. The reading and subsequent identification of the product are performed by analysing the number and intensity of bands (DGGE bands) obtained in each sample of the processed label. This reading is done when confirmation of the composition of the bands of the molecular label is intended (for example: before, during and after marking the products).

An embodiment of the present invention allows the identification and authentication of the product by the reading of the DNA label *in situ* (on the site where the labelled product is). The reading of the label *in situ* provides the user with an additional security guarantee as to the veracity of the results. Sometimes, for legal reasons, the material may not leave the place for analysis or the user needs to prove the authenticity of the product in an expeditious manner *in situ* with witnesses or in the presence of competent authorities, etc....

High accuracy; due to the fact that the DNA molecules are chimeric, it is hardly possible for these to occur naturally. To further increase the complexity of the code, the added amount of each type of chimeric DNA molecule/fraction during the formulation of the molecular label, is also used as information (intensity of the bars) during the reading process thereof. Therefore, the detection and reading of the label of the present invention is highly specific and difficult to forge.

### General description of the invention

The present invention relates to a molecular label and to a marking process of several materials (either biological or non-biological and liquid substances) by using molecular labels containing chimeric deoxyribonucleic acid (DNA) molecules to mark the desired product.

Steps involved in the production and application of the molecular label:

### 1^{st} - Performing model chimeric DNA molecules synthesis

The first step of the molecular label production process involves the production of model chimeric DNA molecules. These molecules are copies of DNA of natural origin and DNA of synthetic origin that will be subsequently produced in large quantities by polymerase chain reaction (PCR) and used in the formulation of the molecular label.

In one embodiment of the present invention, the PCR technic is used to amplify microbial DNA fragments of natural origin (example: biological and environmental samples) using specific primers for certain regions of the genomic DNA and for coupling a GC clamp to the amplified fragments, suitable for DGGE analysis (Heuer et al (2001 - Heuer, H., G. Wieland, J. Schönfeld, A. Schönwälder, N.C.M. Gomes, and K. Smalla 2001 Bacterial community profiling using DGGE or TGGE analysis, p. 177-190. In P. Rouchelle (ed.), Environmental molecular microbiology: protocols and applications Horizon Scientific Press, Wymondham, United Kingdom).

The primers are small DNA fragments previously synthesized *in vitro* by specialized companies (synthetic origin). These are synthesized with complementary nucleotides to the DNA genomic sequences neighbouring the region of interest to be amplified. Due to their easy obtainment and diversity, the primers are often specific to the gene fragments of microbial origin (gene 16S, 18S, *rpoB* and others). Therefore, the aplified DNA consists of copies of the synthetic DNA (primers) and natural genomic DNA (microbial).

The PCR fragments obtained in the preceding step are then connected to a vector (plasmidic DNA molecule) and cloned (Green MR, Sambrook JR (2012) Molecular cloning: a laboratory manual. 4th edition. New York: Cold Spring Harbor Laboratory Press. 2028 p.. After isolation of the clones, a PCR is then carried out to the insert of each clone with specific primers to the sites adjacent to the insertion region of the plasmid. The amplified molecule thus corresponds to copies of the insertion region of the vector and insert. In the present invention this molecule is designated model chimeric DNA molecule that will subsequently be characterized through DGGE technique, produced in large quantities through PCR and used in the formulation of molecular labels.

### 2^{nd} - Performing the Production of the molecular label

In one embodiment of the present invention, in the fourth and fifth steps of the process, the synthesized molecules of model chimeric DNA are amplified by PCR and analysed by denaturing gradient gel electrophoresis (DGGE). Different types of molecules have different mobility in DGGE (bands). Molecules with different mobility (bands in unique positions) are selected for the formulation of the molecular label. At this stage the complexity of the bar code of the molecular label is defined according to the number (kinds of molecules) and intensity (concentration used for each type of molecule) of the bands. This enables the production of labels with unique characteristics according to the needs of each case. For example, a specific label for each company, product or product parts. In general, in terms of mobility in DGGE, a label can contain from 1 to ∼ 100 types (bands) of chimeric DNA molecules.

### 3^{rd} - Performing the application of the molecular label

In one embodiment of the present invention, the label may be applied onto the surface of various materials (either biological or non-biological), added to several food products and liquid substances. For this purpose, depending on the material to be marked, the molecular label may be prepared as a dehydrated or lyophilized solution (ex. ethanol, resins or mainly water). When in a solution, the label may be applied through sprinkling, by droplet or micro droplet deposition, or other mechanism allowing the dispersion of the solution over the material. The lyophilized or dehydrated label may be applied through adhesive substances, resins and or other impregnation methods. The DNA molecules of the label may also be applied in oily substances through its transformation into lipophilic compounds (example: through the use of nanoparticles) or by incorporating the molecular label in phospholipid membranes.

### 4^{th} - Implementation of reading the labels

In one embodiment of the present invention, the reading of the barcode is based in collecting chimeric DNA molecules from previously marked materials (by scraping or smearing), direct amplification of the collected material by PCR reaction (using specific primers) followed by the analysis of PCR product in DGGE. The reading and subsequent identification of the product are performed by analysing the number and intensity of bands (DGGE bands) obtained in each processed label (sample). The reading of the label may also be made from the use of other molecular typing techniques of DNA fragments (example: Polymorphism of single-stranded conformation - SSCP and Polymorphism in the restriction fragments length of the terminal region T-RFLP).

### Brief description of the figures

For an easier understanding of the invention drawings are herein attached, which represent preferred embodiments of the invention which however, do not intend to limit the object of the present invention.
- Figure 1A:: Schematic representation of the synthesis and selection of chimeric DNA molecules.
- Figure 1B:: Schematic representation of the application and detection of the molecular label produced from chimeric DNA molecules.

In a preferred embodiment, the molecular label may be prepared by the following steps:
**1. DNA extraction from** a **natural** or **environmental source,** such as the soil, to be used in the synthesis of chimeric DNA.
**2. Synthesis of DNA fragments of natural origin,** through the use of PCR together with the selected primers to amplify fragments of the 16S rRNA gene of the selected environmental DNA sample, and for the addition of GC clamp, with the use of one of the primers, to the amplified molecules.
**3. Cloning,** the PCR fragments obtained in the previous step are connected to a vector (plasmid DNA molecule), and are used to transform bacterial colonies, which are subsequently isolated and used in the synthesis of the model chimeric DNA molecule.
**4. Synthesis of the model chimeric DNA molecule.** The cloned inserts are amplified by PCR with specific primers for a selected insertion region of the plasmid DNA, a model chimeric DNA molecule being obtained for each insert comprising DNA sequences from plasmid (cloning residue), synthetic (GC clamp and primers) and natural origin (fragments of the selected gene 16S rRNA gene from environmental sample).
**5. Mobility determination of the model chimeric DNA molecule.** The mobility of model chimeric DNA molecules is ascertained by PCR amplification using primers (D2 - SEQ ID NO.5 and R2 - SEQ. ID NO.6) complementary to the insert sequences (cloning residue) being then analysed in terms of mobility in DGGE and selected (molecules with distinct mobility) for the formulation of the molecular label.
**6. Production of the molecular label and application solution.** In this stage the barcode complexity of the molecular label (formulation) is defined depending on the number (types of chimeric DNA molecules) and intensity of DGGE bands. Different types of model chimeric DNA molecules are produced for use in the label. Each label may therefore contain different complexities, conferred by the different number of molecules with distinct mobility, which result in distinct bands. Each label may contain from 1 to -100 types (bands) of model chimeric DNA molecules, thereby defining a certain product, parts of a product, a company, etc.
**7. Application and sampling of molecular label.** The application of the label, in dehydrated or lyophilized solution form may be accomplished in a solid or liquid product, either biological or not, through sprinkling, droplet or micro droplet deposition, or another mechanism enabling the dispersion of the solution over the material, through adhesive substances, resins and other methods of impregnation, when lyophilized in oily substances through its transformation into lipophilic compounds or through the incorporation of the molecular label into phospholipid membranes. Upon reading, the sampling of the molecular label can be done by scraping or a smearing.
**8. Reading of molecular labels:** The reading of the barcode is based on the collection of chimeric DNA molecules, amplification of the material collected by PCR with the use of specific primers followed by the analysis of the PCR product in DGGE or from the use of other techniques for DNA fragment molecular typing. The reading and subsequent identification of the product are performed by analysing the number and intensity of bands (DGGE bands) obtained in each processed sample.

In an embodiment of the present invention, in order to facilitate the localization of the label at the time of reading, the marking site of the product should be defined (example: on the tail of the fish, on the surface of an electronic chip, etc.) and/or the chimeric DNA molecules mixed in dyes or paints should be applied. The molecular label can also be impregnated into a paper label or be integrated with other technologies, such as electronic labels (example: RFID).

In another embodiment of the present invention a faster and simpler detection *in situ* of one or all model molecules used in the molecular label is possible. This is achieved through the use of techniques based on isothermal amplification of nucleic acids (example: Loop-mediated isothermal amplification - LAMP and rolling circle amplification - RCA) and methodological variations thereof (example: types and numbers of primers) (Notomi T, Okayama H, Masubuchi H, Yonekawa T, Watanabe K, Amino N, Haseet T. 2000. Loop-mediated isothermal amplification of DNA Nucleic Acids Res 28:e63; Smith JH, Beals TP. 2013. Detection of Nucleic Acid Targets using Ramified Rolling Circle DNA Amplification: A Single Nucleotide Polymorphism Assay Model. PLoS ONE 8:e65053. doi:10.1371/journal.pone.0065053). However, despite the simple and prompt obtaining of results, these techniques are more expensive than those of PCR and DGGE. The authentication and traceability process of many products may be highly expensive for a company. Perhaps in the future, with the popularization and cost decrease of DNA isothermal amplification techniques, it shall be possible for said techniques to be routinely used in the detection and reading of DNA labels of the present invention.

### Detailed Description of the Invention

The present invention relates to a molecular label and to a process for marking various materials and liquid substances from the use of molecular labels containing chimeric deoxyribonucleic acid molecules (DNA) (Figures 1A and 1B).

### In one of the embodiments of the present invention, the extraction of DNA and synthesis of the model chimeric DNA molecule may be carried out as follows:

a) *DNA Extraction* (step 1). The first step in the process for the production of the molecular label involves the extraction of microbial DNA from natural or environment samples in general (example: microorganism from soil, sediment, water or vegetable samples). In this embodiment of the invention, the DNA used for the synthesis of chimeric DNA molecules was obtained from soil samples according to the protocol previously published by Gomes et al (GOMES, N.C.M., COSTA, R.S., SMALLA, K. (2004) Rapid simultaneous extraction of DNA and RNA from bulk and rhizosphere soil In: Molecular microbial ecology manual. Kowalchuk, G.A., Bruijn, F.J. de, Head, I.M., Akkermans, A.D.L., Elsas, J.D. van. (eds) Kluwer Academic Publishers 10pp. 2 Ed). However, other more recent commercial kits and protocols may also be used.
b) *Synthesis of DNA fragments from natural ori gin* (step 2). Then, the PCR technic is used to amplify the 16S rRNA gene fragments (DNA of natural origin) using primers with GC clamp on the end of one of them. The GC clamp is intended to prevent complete denaturing of the amplified DNA fragment during the DGGE analysis thereof (Muyzer G., De Waal E.C., Uitterlinden A.G. (1993) Profiling of complex microbial populations by denaturing gradient gel electrophoresis analysis of polymerase chain reaction-amplified genes coding for 16S rRNA. Appl. Environ. Micro-biol 59:695-700.).

### In one embodiment of the present invention the following primers were used:

- F968-GC - *SEQ ID* NO.*1*: - 5'-CGCCCGGGGCGCGCCCCGGGCGGGGCGGGGGCACGGGGGGAACGCGAAGAACCTTAC-3' and,
- L1401 - *SEQ ID* NO.*2*: - 5'-CGGTGTGTACAAGGCCCGGGAACG-3'
to obtain (amplification) fragments of the 16S rRNA gene according to the PCR protocol previously published by Heuer et al (2001 - Heuer, H., G., Wieland, J. Schönfeld, A. Schönwälder, N.C.M. Gomes, and K. Smalla. 2001. Bacterial community profiling using DGGE or TGGE analysis, p. 177-190. In P. Rouchelle (ed.), Environmental molecular microbiology: protocols and applications Horizon Scientific Press, Wymondham, United Kingdom.).

However, this invention does not depend exclusively on the gene 16S rRNA for the synthesis of the chimeric DNA molecules. Primers for PCR reaction containing the GC clamp may be used to virtually amplify any part of the microbial genomic DNA, including fragments of other DNA noncoding parts.
c) *Cloning* (step 3). PCR fragments obtained in the previous step (insert) are then linked to plasmid vectors (recombinant plasmids) and processed through the use of a cloning kit (example: pGEM-T kit Easy vector by Promega) or through conventional methodologies (Green MR, Sambrook JR (2012) Molecular cloning: a laboratory manual. 4th edition. New York: Cold Spring Harbor Laboratory Press. 2028 p.). In general, recombinant plasmids are transformed in such step into *Escherichia coli.* Then, the transformed bacteria are selected in a selective culture medium for the bacteria containing the recombinant plasmid. Colonies of bacteria containing the recombinant plasmid are then isolated (pure cultures) and are later used in the synthesis of the model chimeric DNA molecule.
d) *Synthesis of the model chimeric DNA molecule* (step 4). With the purpose of verifying the presence of the insert (cloned DNA fragment) and producing the model chimeric DNA molecule, small aliquots of the host/vector system (clones: colonies of *Escherichia coli* containing recombinant plasmid) are then added in 0.2 mL tubes containing reagents for the PCR and primers:
   - D1 - SEQ. ID NO.3: 5'-GGCCAGTGAATTGTAATACG- 3' and
   - R1 - SEQ. ID NO.4: 5'- CACAGGAAACAGCTATGACC - 3'
   specific for the insertion region of the plasmid DNA pGEM-T, Promega. The amplified DNA fragment comprises part of the insertion region of the vector (the region flanking the insert) and the insert.

Therefore, in this phase the DNA fragment designated in the present invention as model chimeric DNA molecule is obtained; containing DNA sequences of plasmid, synthetic (GC clamp and primers) and natural is obtained (gene 16S rRNA fragment) (Figure 1A). The chimeric DNA molecules obtained at this stage are subsequently characterized through DGGE technique for the formulation of the molecular labels.

*Another form of obtaining the model chimeric DNA molecule may be as follows:* The synthesis of the model chimeric DNA molecule can also be accomplished through the use of PCR, without the need of cloning. DNA fragments of natural origin (see topic 1.a) can be amplified with slightly modified primers to DGGE (see topic 1.b). It is sufficient to add, at the 5' terminal of the primers, a tail of 12 nucleotides randomly arranged with varying proportions of adenine, guanine, thymine and cytosine. The DNA fragments obtained may then be re-amplified (PCR) using miniprimers (Isenbarger TA, Finney M, Rios-Velazquez C, Handelsman J, Ruvkun G. 2008 Miniprimer PCR, the new lens for viewing the microbial world. Appl. Environ. Microbiol. 74:840-849.) additional to the nucleotide sequences of the tails, added to the primers. These primers are then used for the production (see topic 2) and later reading of the model chimeric DNA molecule via PCR-DGGE (see topic 4). Thus, it is possible to produce chimeric DNA molecules without the use of cloning techniques. The disadvantage is due to the fact that this approach often produces more than one chimeric DNA molecule per PCR reaction. Therefore, unlike cloning it does not allow handling each chimeric DNA molecule individually synthesized and consequently offers less options regarding the formulation and standardization of the molecular labels.

### In one embodiment of the present invention the characterization and production of the molecular label may be performed as follows:

a) *Determination of the mobility of the model chimeric* DNA *molecule in DGGE* (step 5). The model chimeric DNA molecules synthesized in the previous step (PCR product) are then submitted to a second cycle of amplification (PCR) using primers D2 - SEQ. ID NO.5: 5'-TAATACGACTCACTALABELGG-3' and R2- SEQ. ID NO.6_: 5'-ATTLABELGTGACACTALABEL-3' complementary to the insertion sequences (cloning residue).
   Then a small aliquot (2-4µl) of the PCR product is analysed in DGGE according to the protocol published by Heuer et al (2001-Heuer, H., G. Wieland, J. Schönfeld, A. Schönwälder, N.C.M. Gomes, and K. Smalla. 2001. Bacterial community profiling using DGGE or TGGE analysis, p. 177-190. In P. Rouchelle (ed.), Environmental molecular microbiology: protocols and applications Horizon Scientific Press, Wymondham, United Kingdom). The band profile of each label may be registered with the aid of a scanner. If needed, the registration and comparison of a large number of labels, the DGGE image may be processed with a specific program for the transformation of the DGGE profiles into densitometry curves. This analysis may be carried out according to the methodology published by Gomes et al (2010 - Gomes N.C.M., Landi L., Smalla K., Nannipieri P., Brookes P.C., Renella G. (2010) Effects of Cd- and Zn-enriched sewage sludge on soil bacterial and fungal communities. Ecotoxicology and Environmental Safety. 73, 6, 1255-63).
   This procedure allows determining the mobility of the model DNA molecule in DGGE. Different types of molecules have different mobility in DGGE (bands). Therefore, molecules with different mobility (bands in unique positions) are selected for the formulation of the molecular label.
b) *Formulation and production of molecular label* (step 6). At this stage, the complexity of the molecular label barcode (formulation) is defined depending on the number (kinds of chimeric DNA molecules) and intensity of the bands (concentration used of each model chimeric DNA molecule). A label may contain from 1 to ∼100 types of model chimeric DNA molecules (bands). Subsequently to the model DNA molecule with different mobility in DGGE having been chosen to compose the molecular label, the band intensity of each molecule is then decided. The intensity of the bands is defined by the concentration of DNA estimated by spectrophotometry used for each chimeric DNA molecule during the preparation of the molecular label.

Subsequently to the above parameters having been set, insert PCR is again carried out with the primers D1 (SEQ. ID NO.3) and R1 (SEQ. ID NO.4) for each selected molecule (clones containing recombinant plasmid) according to the procedure described in topic 1.d. At this stage the necessary amount of different types of model chimeric DNA molecules for the production of the molecular label is defined. Then the chimeric DNA molecules produced in the application solution are mixed (see topic 3.a), observing the concentration or ratio of each molecule according to decisions made during the formulation step of the label. This methodology allows producing labels with unique characteristics according to the needs of each case. For example, a specific label for each company, product or parts of the product. The quantity of labels to be produced shall depend on the quantity and type of the material to be marked.

The profile of DGGE bands should be registered at the conclusion of the production of each label (see topic 2.a).

The reading of the label (DGGE bands) may also be made from the use of other molecular typing techniques of DNA fragments. For example, the single-stranded conformation polymorphism (SSCP) techniques and polymorphisms in the length of the terminal region restriction fragments (T-RFLP (Hayashi K. (1991) PCR-SSCP: a simple and sensitive method for detection of mutations in the genomic DNA. PCR Methods Appl. 1:34-38.; Liu, W.T., T.L. Marsh, H. Cheng, and L.J. Forney. 1997. Characterization of microbial diversity by determining terminal restriction fragment length polymorphisms of genes encoding 16S rRNA. Appl. Environ. Microbiol. 63: 4516-4522.).

### In one embodiment of the present invention the marking of various materials (either biological or non-biological) and reading of the molecular label can be made as follows:

a) *Application of the molecular label* (step 7). The label may be applied onto the surface of various materials, added into various food products onto the surface of biological materials or living organisms. For this purpose, depending on the material to be marked, the molecular label may be prepared and applied as a dehydrated or lyophilized solution (example: 70% ethanol in ultrapure water, resins or only water). When in solution, the label may be applied by sprinkling, by droplet or micro droplet deposition or other mechanism that allows the spreading of the solution over the material. The lyophilized or dehydrated label may be applied as a suspension through adhesive substances, resins and or other methods of impregnation. The DNA molecules of the label may also be applied to oily substances through its transformation into lipophilic compounds (example: through the use of nanoparticles) or by incorporating the molecular label in phospholipid membranes. To facilitate the location of the label upon reading, one must define the marking site of the product (example: at the tail of the fish, on the surface of an electronic chip, etc.) and/or apply the chimeric DNA molecules mixed in colorants or dyes. The molecular label can also be impregnated into a paper label or be integrated with other technologies, such as electronic labels (example: RFID).
   After the application of the labels, samples of the marked products must be collected (at least 5 for each batch) (see topic 4.a for collecting methods) and profiles of DGGE bands should be determined (see topic 2.a). The result is compared with the profiles of the molecular label at the time of its formulation (quality control). The collected samples should be preserved by freezing (-20°C) and if needed they should be used as reference in later analysis of the product labels.
b) *Reading of the label* (step 8). The reading of the barcode (DGGE band profile) of the molecular label starts with collecting a small sample of the marked product by scraping or smearing impregnated with water or other polar or nonpolar solvents (depending on the carrier or form in which the label is fixed). The sample is then dissolved in ultrapure water or TE buffer (Tris-HCl 10mM, EDTA 1mM, pH8) (100-250µl) for 10 minutes at 60°C. Other chemical substances (additives) may be used in the process for dissolving the molecular label, depending on the carrier or form from which the label is undertaken, and on the reagents compatibility with the PCR.

Then, 1µl of the processed sample is used in PCR with primers D2 (SEQ. ID NO.5) /R2 (SEQ. ID NO.6) (see topic 2.a).

PCR consists of the amplification of the copies of chimeric DNA molecules used in the molecular label. The PCR product is then analysed by DGGE (Heuer et al, 2001) (see topic 2.a).

The label reading is based on the analysis of the number and intensity of bands (band profile) obtained in each sample through DGGE (see topic 2.a). The results are then compared with the profile of the reference sample (see topic 3.a). The comparison may be achieved visually or by the analysis *in silico* of the DGGE profiles (see topic 2.a).

If necessary the reading of the label can be done *in situ* (where the product is). All equipment involved in this step may be acquired in its portable or small sized version (example: thermocycler, vertical electrophoresis chamber for DGGE, precast acrylamide gels and accessories). The PCR reagents can be lyophilized in the reaction tubes and be hydrated immediately in place before reading. The remaining reagents can be easily transported to the place of the analysis.

### Examples

### Example 1: Synthesis of chimeric DNA molecule and evaluation of the molecular label for marking samples of dried salted cod

The present example is intended, to show all steps involved in the synthesis, manufacture and use of the molecular label (Figure 1A and 1B). It is further intended to show one use of the molecular label and to evaluate its application in a food product (dried salted cod) according to the present invention. As a conclusion it has been observed that the invention is of easy reproduction, the process for marking and reading the label is fast and simple and the label has shown long durability.

### Steps outlined in Figures 1A and 1B:

**1. DNA Extraction.** In this embodiment of the present invention, the DNA used for the synthesis of chimeric DNA molecules was extracted from soil samples (environmental sample) according to the protocol previously published by Gomes et al (GOMES, N.C.M., COSTA, R.S., SMALLA, K. (2004) Rapid simultaneous extraction of DNA and RNA from bulk and rhizosphere soil in: Molecular microbial ecology manual. Kowalchuk, G.A., Bruijn, F.J. de, Head, I.M., Akkermans, A.D.L., Elsas, J.D. van (eds) Kluwer Academic Publishers. 10pp 2 Ed) (see topic 1.a).
**2. Synthesis of DNA fragments of natural origin.** Then PCR was used together with primers F968-GC - SEQ ID NO.1 and L1401- SEQ ID NO.2 to amplify the fragments of the gene 16S rRNA from the DNA environmental sample. At this stage the primer F968-GC - SEQ ID NO.1 was used to add the GC clamp to the amplified molecules (see topic 1.b).
**3. Cloning.** The PCR fragments obtained in the previous step (insert) were then linked to a vector (plasmid DNA molecule) and cloned using a cloning kit (kit pGEM-T Easy Vector by Promega). Colonies of transformed bacteria (containing the recombinant plasmid) were isolated (pure cultures) and subsequently used in the synthesis of chimeric DNA molecules (see topic 1.c).
**4. Synthesis of model chimeric DNA molecule.** Small aliquots of each clone (positive for the insert) are then added in tubes of 0.2 mL containing reagents for the PCR and specific primers D1 (SEQ ID NO.3) and R1 (SEQ ID NO.4) for the insertion region of plasmid DNA pGEM-T, (Promega). At this stage the DNA fragment designated in the present invention as a model chimeric DNA molecule is obtained; containing DNA sequences of plasmid (cloning residue), synthetic (GC clamp and primers) and natural origin (16S rRNA gene fragments from environmental sample) (see topic 1.d).
**5. Determination of the mobility of the model chimeric DNA molecule in DGGE.** The model chimeric DNA molecules, synthesized in the previous step (PCR product) are then subjected to a second amplification cycle by PCR using primers D2- SEQ ID NO.5 and R2- SEQ ID NO.6 complementary to the insert sequences (cloning residue). Then a small aliquot (2-4µl) of the PCR product is analysed in DGGE according with the protocol published by Heuer et al (2001-Heuer, H., G. Wieland, J. Schönfeld, A. Schönwälder, N.C.M. Gomes, and K. Smalla. 2001. Bacterial community profiling using DGGE or TGGE analysis, p. 177-190. In P. Rouchelle (ed.), Environmental molecular microbiology: Protocols and applications Horizon Scientific Press, Wymondham, United Kingdom.). This procedure allows determining the mobility of the DNA molecule sample in DGGE (see topic 2.a).
**6. Production of the molecular label and application solution.** In this phase, the complexity of the molecular label barcode (formulation) is defined according to the number (types of chimeric DNA molecules) and intensity of DGGE bands. In this example, two molecules with different mobility (bands on sole position) in DGGE have been selected, according to the analysis of DGGE bands in the previous section. These molecules were designated as type 1 and type 2, and were then used in the formulation of the molecular label.
   PCR was then performed for the recombinant plasmids containing selected molecules (inserts for type 1 and 2 chimeric DNA molecules) in 50 µl of reaction with primers D1 (SEQ. ID NO.3) and R1 (SEQ. ID NO.4) (see topic 2.b). The PCR product was then purified by precipitation with ethanol and was reconstituted in 50 µl ultra-pure water. Then the molecular label was prepared from the homogenization of the PCR product obtained for the type 1 and 2 chimeric DNA molecules (∼200 ng each 1:1) in 10 mL application solution (70% ethanol in ultra-pure water).
**7. Application and sampling of molecular label.** The label application was prepared with the aid of a brush, through spreading of ethanol solution containing the label on the caudal fin of 15 pieces of dried salted cod. The marked cod pieces were then maintained under refrigeration (4°C) for a 150 days period. Samples of the label (3 independent reproductions) were obtained from smearing after application and at interims of 50 days with the aid of a swab soaked in 70% ethanol.
**8. Reading of the samples by DGGE.** The swab end corresponding to each sample was then immersed in TE buffer (Tris-HCl 10 mM, EDTA 1mM, pH8) (250 µl) and incubated at 60°C during 10 minutes.

After incubation the sample was homogenized in vortex for 10 seconds. The reading of the molecular label was performed directly from the samples collected in TE through PCR-DGGE technic (see topic 2.a). Figure 1B shows that model chimeric DNA molecules used in the formulation of molecular label (referred to as type 1 and 2) were detected by DGGE 150 days after the marking of the samples (cod 1, 2 and 3). The detected band profile confirms the presence of both types of model chimeric DNA molecules initially used in the formulation of the molecular label. Even keeping the intensity of the bands at equal ratio (1:1) as it was set during the formulation of the molecular label in step 6.
Lisbon, 23 March, 2016

### SEQUENCE LISTING

Primer F968-GC / SEQ. ID NO.1:

5´-CGCCCGGGCGCGCCCCGGCGGGCGGGGGCACGGGGGAACGCGAA
GAACCTTAC-3´

Primer L1401 / SEQ. ID NO.2:
5'-CGGTGTGTACAAGGCCCGGGAACG-3'

Primer D1 / SEQ. ID NO.3:
5'-GGCCAGTGAATTGTAATACG- 3'

Primer R1 / SEQ. ID NO.4:
5'- CACAGGAAACAGCTATGACC - 3'

Primer D2 / SEQ. ID NO.5:
5'-TAATACGACTCACTATAGGG-3'

Primer R2 / SEQ. ID NO.6:
5'-ATTTAGGTGACACTATAG-3'

GC Cl am /SEQ. ID NO.7:
5'-CGCCCGGGGCGCGCCCCGGGCGGGGCGGGGGCACGGGGGG-3'

## Claims

1. Molecular label to mark a substrate comprising:
a mixture of a plurality of DNA molecules, each comprising a GC-clamp DNA fragment as an identifier segment and a microbial DNA fragment as a encoder segment,
wherein at least two of said microbial DNA fragments derive from different microorganisms;
and a carrier for supporting said mixture.

2. Molecular label according to the preceding claim wherein said DNA molecules were obtainable separately.

3. Molecular label according to the preceding claims wherein the size of said microbial DNA fragments is between 200-1800 pb.

4. Molecular label according to the preceding claims wherein each of said fragments has the same number of pair bases.

5. Molecular label according to the preceding claims further comprising primers selected from the following list: SEQ. ID NO.1; SEQ ID NO.2; SEQ. ID NO.3; SEQ. ID NO.4; SEQ. ID NO.5; SEQ. ID NO.6.

6. Molecular label according to the preceding claims wherein the concentration of each one of said DNA molecules is variable.

7. Molecular label according to the preceding claims wherein the substrates are solid or liquid.

8. Molecular label according to the preceding claims wherein the carriers for solid substrates are selected from the following list: epoxy resins, paints, carrier particles, phospholipid membranes.

9. Molecular label according to the preceding claims wherein the carriers for liquid substrates are selected from the following list: aqueous solutions, ethanol, acetone, phospholipid membranes.

10. Molecular label according to the preceding claims wherein it is in dehydrated or lyophilized form.

11. Labelled object comprising the labels according to the preceding claims.

12. Labelled object wherein the object is a food, or medicine, or paper, or clothing, or a watch, or jewellery, or an electronic item.

13. Method for incorporating the molecular label according to the preceding claims in the substrate to be marked comprising the following steps:
• synthesis of each DNA molecule from a plurality of primers and gene fragments selected from one or more microbial DNA molecules, wherein each DNA molecule contains a GC-clamp DNA fragment as an identifier segment and a microbial DNA fragment as a encoder segment;
• mixture of each DNA molecule obtained;
• production of at least one molecular label comprising a mixture of a plurality of DNA molecules, with a certain density conferred by the respective DNA concentration;
• marking the substrate with at least one molecular label.

14. Process according to the preceding claim wherein said DNA molecules are obtainable separately by the cloning step in *Escherichia coli* and preparation of genomic libraries containing different types of inserts.

15. Process according to claim 13 wherein the synthesis of at least one of said DNA fragments comprises the insertion of appropriate primers to sites adjacent to the insertion region of the plasmid.

16. Process according to claims 13-15 wherein said primers are selected from the following list: SEQ. ID NO.1; SEQ. ID NO.2; SEQ. ID NO.3; SEQ. ID NO.4; SEQ. ID NO.5; SEQ. ID NO.6.

17. Process according to claims 13-16 wherein the molecular label is dehydrated, lyophilized, kept in solution form, in nanoparticle form.

18. Process according to claims 13-17 wherein the application of the label in the substrate is performed by sprinkling, by droplet or micro droplet deposition, impregnation with adhesive and/or resins.

19. Process according with claims 13-18 wherein the molecular label further comprises a phospholipid membrane.

20. Identification and/or traceability process of various materials comprising the detection of at least one molecular label according to claims 1-12.

21. Identification and/or traceability process of various materials according to the preceding claim further comprising the step of comparing at least one molecular label with the profile of a reference sample of the molecular label used to mark the material.
Lisbon, 23 March, 2016
